Europäisches Patentamt

European Patent Office

Office européen des brevets

(11) Veröffentlichungsnummer: **0 179 352 B1**

## EUROPÄISCHE PATENTSCHRIFT

(45) Veröffentlichungstag der Patentschrift: **30.12.92**

(51) Int. Cl.⁵: **C07K 5/00**, A61K 37/02

(21) Anmeldenummer: **85112757.1**

(22) Anmeldetag: **08.10.85**

Die Akte enthält technische Angaben, die nach dem Eingang der Anmeldung eingereicht wurden und die nicht in dieser Patentschrift enthalten sind.

(54) **Peptide.**

(30) Priorität: **20.10.84 DE 3438545**

(43) Veröffentlichungstag der Anmeldung:
**30.04.86 Patentblatt 86/18**

(45) Bekanntmachung des Hinweises auf die Patenterteilung:
**30.12.92 Patentblatt 92/53**

(84) Benannte Vertragsstaaten:
**AT BE CH DE FR GB IT LI NL SE**

(56) Entgegenhaltungen:
EP-A- 118 223
EP-A- 0 045 665
EP-A- 0 081 783
EP-A- 0 155 809
EP-A- 0 163 237

CHEMICAL ABSTRACTS, Band 94, 1981, Seite 274, Zusammenfassung Nr. 152461e, Columbus, Ohio, US; C.G. KNIGHT et al.:
"Interaction of dinitrophenyl-pepstatins with human cathepsin D and with anti-dinitrophenyl antibody. Development of potential reagents for the localization in vivo of active proteinases at sites of tissue injury"

(73) Patentinhaber: **MERCK PATENT GESELL-SCHAFT MIT BESCHRÄNKTER HAFTUNG**
**Frankfurter Strasse 250 Postfach 4119**
**W-6100 Darmstadt(DE)**

(72) Erfinder: **Hölzemann, Günter, Dr.**
**Weedring 5**
**W-6104 Seeheim 1(DE)**
Erfinder: **Raddatz, Peter, Dr.**
**Grafenstrasse 37**
**W-6100 Darmstadt(DE)**
Erfinder: **Jonczyk, Alfred, Dr.**
**Jungfernstrasse 30**
**W-6100 Darmstadt(DE)**
Erfinder: **Schmitges, Claus J., Dr.**
**Karolingerstrasse 6**
**W-6114 Gross-Umstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Peptide der Formel I

X-Z-NH-CHR-CHOH-CH$_2$-CO-NH-CHR$^1$-CH$_2$-NH-CHY-C$_n$H$_{2n}$-R$^2$   I

worin

| | |
|---|---|
| X | A-O-CO- oder C$_6$H$_5$ CH$_2$-O-CO-, |
| Z | Gly, Phe-Ala, Phe-Arg, Phe-Asn, Phe-Gln, Phe-Gly, Phe-His, Phe-Leu, Phe-Lys, Phe-Met, Phe-Nle, oder Phe-Orn, |
| R | Isobutyl oder Cyclohexylmethyl |
| R$^1$ | Isopropyl, Isobutyl oder sek-Butyl, |
| Y | H oder -CH$_2$OR$^3$, |
| R$^2$ | Phenyl oder p-Hydroxyphenyl, |
| R$^3$ | H oder A, |
| A | Alkyl mit 1 - 6 C-Atomen und |
| n | 0, 1 oder 2 bedeuten, |

sowie deren Salze.

Ähnliche Verbindungen sind aus der EP-A-77028 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250 - 256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind wesentlich höhere Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste -NH-CHR-CO- (worin R die für jede Aminosäure bekannte spezifische Bedeutung hat) folgender Aminosäuren:

| | |
|---|---|
| Ala | Alanin |
| Arg | Arginin |
| Asn | Aparagin |
| Gln | Glutamin |
| Gly | Glycin |
| His | Histidin |
| Ile | Isoleucin |
| Leu | Leucin |
| Lys | Lysin |
| Met | Methionin |
| Nle | Norleucin (2-Aminohexansäure) |
| Orn | Ornithin |
| Phe | Phenylalanin |
| Sta | Statin (3-Hydroxy-4-amino-6-methylheptansäure) |
| Tyr | Tyrosin |
| Val | Valin. |

Die Bezeichnung "-red-" steht für eine reduzierte Peptidbindung (-CH$_2$-NH- statt -CO-NH).

Ferner bedeuten nachstehend:

| | |
|---|---|
| BOC | tert.-Butoxycarbonyl |
| BOM | Benzyloxymethyl |
| CBZ | Benzyloxycarbonyl |
| DCC | Dicyclohexylcarbodiimid |
| DMF | Dimethylformamid |
| DNP | 2,4-Dinitrophenyl |
| FMOC | 9-Fluorenylmethoxycarbonyl |
| HOBt | 1-Hydroxybenzotriazol |
| OMe | Methylester |
| POA | Phenoxyacetyl. |

Sofern die genannten Aminosäuren in mehreren enantiomeren Formen auftreten können, sind vor- und nachstehend, z. B. als Bestandteil der Verbindungen der Formel I, alle diese Formen und auch ihre Gemische (z. B. die DL-Formen) eingeschlossen. Die L-Formen mit S-Konfiguration sind bevorzugt. Sofern nachstehend einzelne Verbindungen aufgeführt sind, beziehen sich die Abkürzungen dieser Aminosauren jeweils auf die L-Form, sofern nicht ausdrücklich etwas anderes angegeben ist; so bedeutet "Sta" in den nachstehenden Einzelverbindungen den Rest der 3S-Hydroxy-4S-amino-6-methylheptansäure.

In den vorstehenden Formeln hat A 1 - 6, vorzugsweise 1,2,3 oder 4 C-Atome. A bedeutet vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Die Gruppe -NH-CHR$^1$-CH$_2$- ist ein "reduzierter" Aminosäurerest. R$^1$ ist daher vorzugsweise sek.-Butyl (abgeleitet von Isoleucin) oder Isobutyl (Leucin), ferner Isopropyl (Valin). Das chirale Zentrum -CHR$^1$- kann in verschiedenen enantiomeren Formen vorliegen; die S-Formen der zugrundeliegenden "reduzierten" Aminosäuren sind dabei bevorzugt. Wenn nichts anderes angegeben ist, liegen die nachstehend angegebenen Einzelverbindungen immer in der Form vor, die bezüglich der Gruppe -CHR$^1$- der S-Form der R$^1$ entsprechenden "reduzierten" Aminosäure entspricht.

R$^3$ bedeutet vorzugsweise A, insbesondere Methyl, Ethyl, Propyl, Isopropyl, Butyl, Isobutyl oder tert.-Butyl.

X bedeutet vorzugsweise Alkoxycarbonyl wie BOC oder CBZ.

Z bedeutet insbesondere die Gruppe Phe-His, ferner bevorzugt die Gruppen Phe-Ala, Phe-Arg, Phe-Asn, Phe-Gln, Phe-Gly, Phe-Ile, Phe-Leu, Phe-Lys, Phe-Met, Phe-Nle, Phe-Orn.

Y ist vorzugsweise H, oder Hydroxymethyl.

Die Gruppierung -NH-CHY-C$_n$H$_{2n}$-R$^2$ ist vorzugsweise von Aminosäuren abgeleitet, deren COOH-Gruppe in reduzierter Form vorliegt. Einige bevorzugte Gruppierungen -NH-CHY-C$_n$H$_{2n}$-R$^2$ sind dementsprechend:

-NH-CH(CH$_2$OH)-CH$_2$C$_6$H$_5$ ("Phe-ol")

-NH-CH(CH$_2$OH)-CH$_2$-p-C$_6$H$_4$OH ("Tyr-ol").

Die chiralen Zentren -CHY- dieser Gruppierungen können in verschiedenen enantiomeren Formen vorliegen; die S-Formen der zugrundeliegenden Aminosäuren sind dabei bevorzugt. Wenn nichts anderes angegeben ist, liegen die nachstehend angegebenen Einzelverbindungen bezüglich der Gruppe -CHY- immer in der Form vor, die der S-Form der Y und R$^2$ entsprechenden Aminosäure entspricht.

Weitere bevorzugte Bedeutungen der Gruppierung -NH-CHY-C$_n$H$_{2n}$-R$^2$ sind insbesondere -NH-CH$_2$-C$_n$H$_{2n}$-R$^2$, z.B. -NH-CH$_2$-R$^2$, -NH-(CH$_2$)$_2$-R$^2$ oder -NH-(CH$_2$)$_3$-R$^2$, im einzelnen insbesondere 2-Phenylethylamino, 2-(p-Hydroxyphenyl)-ethylamino, 3-Phenylpropylamino.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Gegenstand der Erfindung ist ferner ein Verfahren zur Herstellung eines Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt, oder daß man eine Verbindung, die der Formel I entspricht, aber an Stelle von H-Atomen eine oder mehrere zusätzliche hydrogenolytisch abspaltbare Gruppen und/oder -C-C- und/oder -C-N- und/oder -C-O-Bindungen enthält, reduziert, und daß man gegebenenfalls in einer Verbindung der Formel I eine Estergruppe zu einer Hydroxymethylgruppe reduziert und/oder eine Verbindung der Formel I durch Behandeln mit einer Säure oder Base in eines ihrer Salze überführt.

Die Verbindungen der Formel I und auch die Ausgangsstoffe zu ihrer Herstellung werden im übrigen nach an sich bekannten Methoden hergestellt, wie sie in der Literatur (z. B. in Standardwerken wie Houben-Weyl, Methoden der Organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Vorzugsweise werden die Verbindungen der Formel I erhalten, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere diejenigen der Formel II

X-Z-NH-CHR-CHOH-CH$_2$-CO-NH-CHR$^1$-CH$_2$-NQ-CHY-C$_n$R$_{2n}$-R$^2$     II

worin

Q eine Aminoschutzgruppe bedeutet.

Ferner sind Ausgangsstoffe bevorzugt, die an Stelle des H-Atoms in 1-Stellung eines Imidazolrings eine Aminoschutzgruppe tragen.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acylgruppen, ferner unsubstituierte oder substituierte Aryl-(z. B. 2,4-Dinitrophenyl) oder Aralkylgruppen (z. B. Benzyl, 4-Nitrobenzyl, Triphenylmethyl), ferner insbesondere Benzyloxymethyl (BOM). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind CBZ, FMOC, Benzyl, Acetyl und BOM.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Methylenchlorid, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50 °, vorzugsweise arbeitet man zwischen 15 und 30 ° (Raumtemperatur).

Die BOC-Gruppe kann z. B. bevorzugt mit 40 %iger Trifluoressigsäure in Methylenchlorid oder mit etwa 3 bis 5 n HCl in Dioxan bei 15-30 ° abgespalten werden, die FMOC-Gruppe mit einer etwa 5- bis 20%igen Lösung von Dimethylamin, Diethylamin oder Piperidin in DMF bei 15-30 °. Eine Abspaltung der DNP-Gruppe gelingt z. B. auch mit einer etwa 3- bis 10%igen Lösung von 2-Mercaptoethanol in DMF/Wasser bei 15-30 °.

Hydrogenolytisch entfernbare Schutzgruppen (z. B. CBZ, BOM oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Wasserstoffdonatoren sind auch Salze der Ameisensäure geeignet, vorzugsweise Ammoniumformiat. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100 ° und Drucken zwischen etwa 1 und 200 bar, bevorzugt bei 20-30 ° und 1-10 bar durchgeführt. Eine Hydrogenolyse der CBZ-Gruppe gelingt z. B. gut an 5- bis 10%igem Pd-C in Methanol bei 20-30 °.

Die Verbindungen der Formel I sind auch erhältlich durch Reduktion entsprechender Verbindungen, die an stelle von H-Atomen hydrogenolytisch abspaltbare Gruppen und/oder zusätzliche -C-C- und/oder -C-N- und/oder -C-O-Bindungen enthalten.

Als Ausgangsstoffe für die Reduktion eignen sich z.B. Schiffsche Basen der Formel $X-Z-NH-CHR-CHOH-CH_2-CO-NH-CHR^1-CH = N-CHY-C_nH_{2n}-R^2$, die aus Aldehyden der Formel $X-Z-NH-CHR-CHOH-CH_2-CO-NH-CHR^1-CHO$ und Aminen der Formel $H_2N-CHY-C_nH_{2n}-R^2$ hergestellt werden können. Diese können vorteilhaft katalytisch unter den vorstehend genannten Bedingungen zu Verbindungen der Formel I hydriert werden, z. B. an einem Edelmetall-Katalysator wie Pd-Kohle in einem inerten Lösungsmittel wie Methanol bei etwa 20 - 30 ° und 1 - 10 bar.

Weiterhin können z. B. Ketoverbindungen der Formel $X-Z-NH-CHR-CO-CH_2-CO-NH-CHR^1-CH_2-NH-CHY-C_nH_{2n}-R^2$ zu Verbindungen der Formel I reduziert werden, beispielsweise mit einem komplexen Metallhydrid wie $NaBH_4$ in einem inerten Lösungsmittel wie Methanol bei Temperaturen zwischen etwa -10 und +30 °.

Weiterhin ist es z. B. möglich, einen Ester, welcher sonst der Formel I entspricht, aber an Stelle von Y eine COOA-Gruppe enthält, zur entsprechenden Hydroxymethylverbindung der Formel I (Y = $CH_2OH$) zu reduzieren, z. B. mit Diisobutylaluminiumhydrid in einem inerten Lösungsmittel wie Dichlormethan bei Temperaturen zwischen -60 und +30 °.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Eine Säure der Formel I kann durch Umsetzung mit einer Base in eines ihrer physiologisch unbedenklichen Metall- bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze, z. B. die Dimethyl-, Diethyl- oder Diisopropylammonium-, Monoethanol-, Diethanol- und Triethanolammonium-, Cyclohexylammonium-, Dicyclohexylammonium- und Dibenzylethylendiammmoniumsalze, weiterhin z. B. Salze mit N-Methyl-D-glucamin oder mit basischen Aminosäuren wie Arginin oder Lysin.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluor-chlor-kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten, insbesondere der reninabhängigen Hypertension oder des Hyperaldosteronismus verwendet werden. Dabei werden die erfindungsgemäßen Substanzen in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt auf pH 6 - 8 ein, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Man löst 1 g BOC-Phe-His(DNP)-Sta-N-[1-(N-(1-hydroxymethyl-2-phenyl-ethyl)-N-FMOC-aminomethyl)-2-methyl-butyl]-amid ["BOC-Phe-His-(DNP)-Sta-Ile-red-N(FMOC)-Phe-ol"; F. 135-138 °; erhältlich durch Kondensation von BOC-Ile-OH mit H-Phe-OMe in Gegenwart von DCC/HOBt in $CH_2Cl_2$ zu BOC-Ile-Phe-OMe (F. 118 °), Reduktion mit Natrium-bis-(2-methoxyethoxy)aluminiumhydrid in Benzol zu 2-(2-BOC-amino-3-methyl-pentylamino)-3-phenylpropanol ("BOC-Ile-red-Phe-ol"; F. 78 °. Umsetzung mit FMOC-Cl/$NaHCO_3$ in Aceton zu 2-[N-FMOC-N-(2-BOC-amino-3-methylpentyl)-amino]-3-phenylpropanol ["BOC-Ile-red-N(FMOC)-Phe-ol"; Öl], Abspaltung der BOC-Gruppe mit 2n HCl in Dioxan zu 2-[N-FMOC-N-(2-amino-3-methylpentyl)amino]-3-phenylpropanol ["Ile-red-N(FMOC)-Phe-ol"], Reaktion mit BOC-Sta-OH/DCC/HOBt zu "BOC-Sta-Ile-red-N(FMOC)-Phe-ol", Spaltung zu "Sta-Ile-red-N(FMOC)-Phe-ol", Reaktion mit BOC-His(DNP)-OH/DCC/HOBt zu "BOC-His(DNP)-Sta-Ile-red-N(FMOC)-Phe-ol", Spaltung zu "His(DNP)-Sta-Ile-red-N(FMOC)-Phe-ol" und Kondensation mit BOC-Phe-OH/DCC/HOBt] in 20 ml DMF, versetzt bis zur Trübung mit Wasser, gibt $NaHCO_3$-Lösung bis pH 8,5 und dann tropfenweise 10 ml 2-Mercaptoethanol hinzu. Nach 2std. Rühren bei 20 ° wird die Reaktionslösung in Wasser eingerührt und mit Ethylacetat extrahiert. Nach dem Trocknen des Extrakts über $Na_2SO_4$ dampft man ein und löst das so erhaltene ölige "BOC-Phe-His-Sta-Ile-red-N-(FMOC)-Phe-ol" in einem Gemisch aus 6 ml Piperidin und 15 ml DMF. Man rührt 1 Std., dampft ein, verreibt den Rückstand mit Ether, reinigt durch Chromatographie an Sephadex LH 20 und erhält BOC-Phe-His-Sta-N-[1-(N-(1-hydroxymethyl-2-phenyl-ethyl)-aminomethyl)-2-methylbutyl]-amid ("BOC-Phe-His-Sta-Ile-red-Phe-ol"), F. 100 - 103 °.

Analog erhält man aus den entsprechenden His(DNP)-N(FMOC)-derivaten:
BOC-Phe-His-Sta-N-[1-(N-(2-phenylethyl)-aminomethyl)-3-methylbutyl]-amid ("BOC-Phe-His-Sta-Leu-red-$NHCH_2CH_2C_6H_5$"), F. 115 - 119 °
BOC-Phe-His-Sta-N-[1-(N-(2-phenylethyl)-aminomethyl)-2-methylbutyl]-amid ("BOC-Phe-His-Sta-Ile-red-$NHCH_2CH_2C_6H_5$"), F. 110 - 115 °
BOC-Phe-His-Sta-N-[1-(N-(1-hydroxymethyl-2-phenyl-ethylaminomethyl)-3-methylbutyl]-amid ("BOC-Phe-His-Sta-Leu-red-Phe-ol"), F. 113 - 115 °
BOC-Phe-His-Sta-N-[1-(N-(1-hydroxymethyl-2-phenyl-ethyl)-aminomethyl)-2-methylpropyl]-amid ("BOC-Phe-His-Sta-Val-red-Phe-ol"), F. 180 ° (Zers.)
BOC-Phe-His-Sta-N-[1-(N-(2-phenylethyl)-aminomethyl)-2-methylpropyl]-amid ("BOC-Phe-His-Sta-Val-red-$NHCH_2CH_2C_6H_5$"), F. 170 - 173 °
BOC-Phe-His-Sta-N-[1-(N-(3-phenylpropyl)-aminomethyl)-3-methylbutyl]-amid ["BOC-Phe-His-Sta-Leu-red-NH-$(CH_2)_3$-$C_6H_5$"], F. 90 - 93 °
BOC-Phe-Ala-Sta-Ile-red-Phe-ol
BOC-Phe-Arg-Sta-Ile-red-Phe-ol, F. 84-90 °
BOC-Phe-Asn-Sta-Ile-red-Phe-ol, F. 152-155 °
BOC-Phe-Gln-Sta-Ile-red-Phe-ol, F. 183-185 °
BOC-Phe-Gly-Sta-Ile-red-Phe-ol
BOC-Phe-Leu-Sta-Ile-red-Phe-ol
BOC-Phe-Lys-Sta-Ile-red-Phe-ol
BOC-Phe-Met-Sta-Ile-red-Phe-ol
BOC-Phe-Nle-Sta-Ile-red-Phe-ol, F. 112-118 °
BOC-Phe-Orn-Sta-Ile-red-Phe-ol

Beispiel 2

Eine Lösung von 1 g "BOC-Phe-His(BOM)-Sta-Ile-red-N($CH_2C_6H_5$)-Phe-ol" [erhältlich aus "BOC-Ile-red-Phe-ol" durch N-Benzylierung und Abspaltung der BOC-Gruppe zu "Ile-red-N($CH_2C_6H_5$)-Phe-ol" und nachfolgenden stufenweisen Aufbau der Peptidkette analog Beispiel 1] in 50 ml Methanol wird an 1 g $Pd(OH)_2$ bei 20 ° und 3 bar 4 Std. lang hydriert. Man filtriert, dampft ein und erhält "BOC-Phe-His-Sta-Ile-red-Phe-ol",F. 100 - 103 °.

Beispiel 3

Eine Lösung von 1 g BOC-Phe-His-Sta-N-[1-(N-(1-hydroxymethyl-2-phenylethyl)-iminomethyl)-2-methyl-butyl]-amid (erhältlich durch Kondensation von BOC-Phe-His-Sta-Ile-al mit Phe-ol) in 50 ml Methanol wird an 1 g 5 %ig. Pd-C bei 20 ° und 1 bar bis zum Stillstand der $H_2$-Aufnahme hydriert. Man filtriert, dampft ein und erhält "BOC-Phe-His-Sta-Ile-red-Phe-ol", F. 100 - 103 °.

Beispiel 4

Eine Lösung von 777 mg 3-Oxo-4S-(BOC-Phe-His-amino)-6-methylheptansäure-N-[1-(N-(1-Hydroxymethyl-2-phenyl-ethyl)-aminomethyl)-2-methyl-butyl]-amid [erhältlich durch Reaktion von "Ile-red-N(FMOC)-Phe-ol" mit 3-Oxo-4-BOC-amino-6-methylheptansäure zu 3-Oxo-4S-BOC-amino-6-methylheptansäure-[1-(N-(1-hydroxymethyl-2-phenyl-ethyl)-N(FMOC)-aminomethyl)-2-methyl-butyl]-amid, Abspaltung der BOC-Gruppe zur 3-Oxo-4S-amino-verbindung, Reaktion mit BOC-His-(DNP)-OH zu 3-Oxo-4S-(BOC-His(DNP)-amino)-6-methyl-heptansäure-[1-(N-(1-hydroxymethyl-2-phenylethyl)-N(FMOC)-aminomethyl)-2-methyl-butyl]-amid, Abspaltung der BOC-Gruppe zur 3-Oxo-4S-(His(DNP)-amino)-verbindung, Kondensation mit BOC-Phe-OH zu 3-Oxo-4S-(BOC-Phe-His-amino)-6-methylheptansäure-[1-(N-(1-hydroxymethyl-2-phenyl-ethyl)-FMOC-aminomethyl)-2-methyl-butyl]-amid und Abspaltung der DNP- sowie der FMOC-Gruppe analog Beispiel 1] in 40 ml Methanol wird bei 0 ° mit 200 mg NaBH₄ versetzt. Man rührt 2 Std., gibt Salzsäure zur Zerstörung des überschüssigen NaBH₄ hinzu, arbeitet wie üblich auf und erhält ein Gemisch zweier Stereoisomerer, aus denen sich "BOC-Phe-His-Sta-Ile-red-Phe-ol",F. 100 - 103 °, chromatographisch (Kieselgel) abtrennen läßt.

Beispiel 5

Eine Lösung von 821 mg "BOC-Phe-His-Sta-Ile-red-Phe-OEt" in 45 ml $CH_2Cl_2$ wird bei -50 ° mit 350 mg Diisobutylaluminiumhydrid versetzt und 1 Std. gerührt. Nach üblicher Aufarbeitung erhält man "BOC-Phe-His-Sta-Ile-red-Phe-ol", F. 100 - 103 °.

Die nachstehenden Beispiele betreffen pharmazeutische Zubereitungen.

Beispiel A: Injektionsgläser

Eine Lösung von 1 kg Phe-His-Sta-Ile-red-Phe-ol-hydrochlorid und 50 g Dinatriumhydrogenphosphat in 30 l zweifach destilliertem Wasser wird mit 2n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

Beispiel B: Suppositorien

Man schmilzt ein Gemisch von 500 g BOC-Phe-His-Sta-Ile-red-Phe-ol mit 100 g Sojalecithin und 1400 g Kakaobutter, gießt in Formen und läßt erkalten. Jedes Suppositorium enthält 500 mg Wirkstoff.

**Patentansprüche**

1.  Peptide der Formel I

X-Z-NH-CHR-CHOH-$CH_2$-CO-NH-$CHR^1$-$CH_2$-NH-CHY-$C_nH_{2n}$-$R^2$     I

worin

| | |
|---|---|
| X | A-O-CO- oder $C_6H_5CH_2$-O-CO-, |
| Z | Gly, Phe-Ala, Phe-Arg, Phe-Asn, Phe-Gln, Phe-Gly, Phe-His, Phe-Leu, Phe-Lys, Phe-Met, Phe-Nle oder Phe-Orn, |
| R | Isobutyl oder Cyclohexylmethyl, |
| $R^1$ | Isopropyl, Isobutyl oder sek.-Butyl, |
| Y | $H_1$ oder -$CH_2OR^3$, |
| $R^2$ | Phenyl oder p-Hydroxyphenyl, |
| $R^3$ | H oder A, |
| A | Alkyl mit 1 - 6 C-Atomen und |
| n | 0, 1 oder 2 bedeuten, |

sowie deren Salze.

2.
a)     BOC-Phe-His-Sta-N-[1-(N-(1-hydroxymethyl-2-phenyl-ethyl)-aminomethyl)-2-methylbutyl]-amid;
b)  BOC-Phe-His-Sta-N-[-1-(N-(2-phenyl-ethyl)-aminomethyl)-2-methylbutyl]-amid.

3.  Verfahren zur Herstellung eines Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Verbindung, die der Formel I entspricht, aber an Stelle von H-Atomen eine oder mehrere zusätzliche hydrogenolytisch abspaltbare Gruppen und/oder -C-C- und/oder -C-N- und/oder -C-O-Bindungen enthält, reduziert und daß man gegebenenfalls in einer Verbindung der Formel I eine Estergruppe zu einer Hydroxymethylgruppe reduziert und/oder eine Verbindung der Formel I durch Behandeln mit einer Säure oder Base in eines ihrer Salze überführt.

4.  Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5.  Pharmazeutische Zubereitung, gekennzeichnet durch einen Gehalt an mindestens einer Verbindung der Formel I und/oder einem ihrer physiologisch unbedenklichen Salze.

6.  Verbindungen der Formel I und/oder ihre physiologisch unbedenklichen Salze zur Bekämpfung von Krankheiten.

7. Verwendung von Verbindungen der Formel I und/oder ihrer physiologisch unbedenklichen Salze zur Herstellung von Arzneimitteln.

## Claims

1. Peptides of the formula I

X-Z-NH-CHR-CHOH-CH$_2$-CO-NH-CHR$^1$-CH$_2$-NH-CHY-C$_n$H$_{2n}$-R$^2$    I

in which
- X        is A-O-CO- or C$_6$H$_5$CH$_2$-O-CO-,
- Z        is Gly, Phe-Ala, Phe-Arg, Phe-Asn, Phe-Gln, Phe-Gly, Phe-His, Phe-Leu, Phe-Lys, Phe-Met, Phe-Nle or Phe-Orn,
- R        is isobutyl or cyclohexylmethyl,
- R$^1$      is isopropyl, isobutyl or sec.-butyl,
- Y        is H or -CH$_2$OR$^3$,
- R$^2$      is phenyl or p-hydroxyphenyl,
- R$^3$      is H or A,
- A        is alkyl having 1 to 6 C atoms and
- n        is 0, 1 or 2,

and their salts.

2.
a) BOC-Phe-His-Sta-N-[1-(2-hydroxymethyl-2-phenyl-ethyl-aminomethyl)-2-methylbutyl]-amide;
b)     BOC-Phe-His-Sta-N-[-1-(N-(2-phenyl-ethyl)-aminomethyl)-2-methylbutyl]amide.

3. Process for the preparation of a peptide of the formula I and of its salts, characterised in that it is liberated from one of its functional derivatives by treatment with a solvolysing or hydrogenolysing agent, or in that a compound which corresponds to the formula I but in place of H atoms contains one or more additional groups which can be eliminated by hydrogenolysis and/or -C-C- and/or -C-N- and/or -C-O- linkages is reduced, and in that, where appropriate, in a compound of the formula I an ester group is reduced to a hydroxymethyl group, and/or a compound of the formula I is converted into one of its salts by treatment with an acid or base.

4. Process for the preparation of pharmaceutical formulations, characterised in that a compound of the formula I and/or one of its physiologically acceptable salts is converted into a suitable administration form together with at least one solid, liquid or semi-liquid vehicle or auxiliary and, where appropriate, in combination with one or more other active compound(s).

5. Pharmaceutical formulation characterised by containing at least one compound of the formula I and/or one of its physiologically acceptable salts.

6. Compounds of the formula I and/or their physiologically acceptable salts for controlling diseases.

7. Use of compounds of the formula I and/or their physiologically acceptable salts for the preparation of medicaments.

## Revendications

1. Peptides de formule I

X-Z-NH-CHR-CHOH-CH$_2$-CO-NH-CHR$^1$-CH$_2$-NH-CHY-C$_n$H$_{2n}$-R$^2$    I

dans laquelle
- X        représente A-O-CO- ou C$_6$H$_5$CH$_2$-O-CO-,
- Z        représente Gly, Phe-Ala, Phe-Arg, Phe-Asn, Phe-Gln, Phe-Gly, Phe-His, Phe-Leu, Phe-Lys, Phe-Met, Phe-Nle ou Phe-Orn,
- R        représente un groupe isobutyle ou un groupe cyclohexylméthyle,
- R$^1$      représente un groupe isopropyle, un groupe isobutyle ou un groupe sec.-butyle,
- Y        représente H ou -CH$_2$OR$^3$,
- R$^2$      représente un groupe phényle ou un groupe p-hydroxyphényle,
- R$^3$      représente H ou A,
- A        représente un groupe alkyle contenant de 1 à 6 atomes de carbone et
- n        représente 0, 1 ou 2,

ainsi que leurs sels.

2.
a)          BoC-Phe-His-Sta-N-[1-(N-(1-hydroxyméthyl-2-phényléthyl)-aminométhyl)-2-méthylbutyl]-amide;
b)     BOC-Phe-His-Sta-N-[1-(N-(2-phényléthyl)-aminométhyl)-2-méthylbutyl]-amide.

3. Procédé pour la préparation d'un peptide de formule I, ainsi que de ses sels, caractérisé en ce qu'on le libère à partir d'un de ses dérivés fonctionnels par traitement avec un agent de solvolyse ou d'hydrogénolyse, ou bien en ce qu'on réduit un composé qui correspond à celui de la formule I, mais qui contient, à la place des atomes H, un ou plusieurs groupes supplémentaires aptes à être séparés par hydrogénolyse et/ou des liaisons -C-C- et/ou -C-N- et/ou -C-O-, et en ce qu'éventuellement, dans un composé de formule I, on réduit un groupe ester en un groupe hydroxyméthyle et/ou on transforme un composé de formule I en l'un de ses sels par traitement avec un acide ou une base.

4. Procédé pour la mise au point de préparations pharmaceutiques, caractérisé en ce qu'on amène à une forme posologique appropriée, un composé de formule I et/ou un de ses sels physiologiquement acceptables conjointement avec au moins une substance de support au auxiliaire solide, liquide ou semi-liquide et éventuellement en combinaison avec une ou plusieurs autres substances actives.

5. Préparation pharmaceutique caractérisée par une teneur en au moins un composé de formule I et/ou en l'un de ses sels physiologiquement acceptables.

6. composés de formule I et/ou leurs sels physiologiquement acceptables pour lutter contre des maladies.

7. Utilisation des composés de formule I et/ou de leurs sels physiologiquement acceptables pour la préparation de médicaments.